# EUROPEAN PATENT SPECIFICATION

(11) **EP 3 852 704 B1**
(45) Date of publication and mention of the grant of the patent: **01.11.2023**
(21) Application number: 19787347.4
(22) Date of filing: 20.09.2019
(51) Int. Cl.: A61F 9/04, G10K 11/178

(54) **EAR DEVICE AND EAR-AND-EYE MASK WITH NOISE ATTENUATION AND GENERATION**
KOPFHÖRER UND OHR-UND-AUGENMASKE MIT GERÄUSCHDÄMPFUNG UND ERZEUGUNG
ÉCOUTEUR ET MASQUE POUR LES YEUX ET LES OREILLES AVEC ATTÉNUATION ET GÉNÉRATION DE BRUIT

(30) Priority: 20.09.2018 GB 201815368
(43) Date of publication of application: 28.07.2021
(73) Proprietor: Turner-Fernback, Deborah Carol, 10719 Berlin (DE)
(72) Inventor: Turner-Fernback, Deborah Carol, 10719 Berlin (DE)
(74) Representative: Doherty, William
(86) International application number: PCT/GB2019/052654
(87) International publication number: WO 2020/058730

(56) References cited:
- EP-B1- 1 438 709
- EP-B1- 3 123 613
- WO-A1-2017/122091
- WO-A1-2017/196453
- US-A1- 2007 160 251
- US-A1- 2011 209 273
- US-B1- 7 065 219
- US-B1- 7 466 838

## Description

The present invention relates to an ear device which may be part of an ear-and-eye mask, preferably but not necessarily exclusively for use in creating optimum napping conditions for a user, which may be used in combination with a control application on an external device. The invention also relates to a method of creating enhanced napping conditions for a user.

Studies have proven that daytime napping has medical benefits for individuals, in particular for heart health, brain function, and efficient digestion, which is essential for a strong immune system. Napping also improves skin condition and helps to combat obesity by reducing ghrelin levels in the stomach, the hormones which stimulate appetite. Without a nap, Magnetic Resonance Imaging (MRI) scans have shown that brain activity declines as the day progresses.

Noise is also a cause for increased stress levels by activation of the amygdala, an almond shaped structure in the brain, causing release of the stress hormone cortisol, with its resultant health implications.

However, particularly in urban environments, there is significant noise pollution which is detrimental to napping, and it is anticipated that by 2050, 66% of the world's population will be living in cities. Environmental noise, from things like road traffic, trains, planes and wind turbines, is a significant policy issue for the World Health Organization. Research in Europe suggests that noise disturbance can cause real health side effects, and the WHO estimates Western Europeans lose 1 million years of healthy life due to traffic-related noise.

Napping is best undertaken in accordance with the changes in the stages of the sleep cycle and an individual's circadian rhythm, that is, the body's natural 24 hour cycle. For the majority of individuals, this means that the nap is best taken in the late morning or early afternoon, typically for a period of between 10 and 90 minutes. Shift workers, however, may have different cycles, and may nap at later times.

It has been shown that a 10 to 20 minute nap can be sufficient to clear out short-term memories, freeing up the brain for other activities, whilst a 20 to 40 minute nap allows the individual to move through the first two stages of a sleep cycle. This will slow the heart rate, enhance both alertness and concentration, and elevate the individual's mood. Between 40 and 60 minutes, an individual will move into the latter two stages of the sleep cycle when the brain waves slow down significantly and restorative sleep takes place, which allows the brain to make completely new connections, and in addition to the above benefits, significantly increases creative and problem solving skills. Between 60 and 90 minutes, the fifth and last stage of the sleep cycle, the sleeper moves into REM sleep, when brain activity is heightened again and dreams occur. The cycle then repeats. Some researchers have challenged the benefits and importance of REM sleep with mixed views. Most say that REM sleep is essential for making and retaining memories and emotion regulation, whilst others state that REM sleep is when nightmares and traumatic recurring dreams occur, and that the absence thereof is proven to reduce clinical depression.

After an individual has passed from the theta waves of the first two stages of sleep to the slower and higher amplitude delta waves of the third and fourth stages of sleep, the brain is more proficient at blocking external distractions. However, for shorter naps, covering the first two sleep stages, there is a greater risk of external factors waking the individual, which can make napping difficult.

To improve the individual's capacity to nap, blocking out both visual and audible stimuli is very important. In darkness, the body releases melatonin, the hormone that prepares the body to sleep, and in silence, the default mode network of the brain is accessed, improving the ability to process and file thoughts and to think deeply and creatively.

There are no ear devices available which create the necessary silence required for successful napping. Passive products are unable to attenuate noise across the frequency spectrum as successfully, and other available products focus on pairing and streaming audio, and/or in some case stripping ambient noise from the desired music or audio to be played into the ear.

An improved sleep product would be of immediate use in societies which already adopt biphasic sleep habits dividing their sleep into two chunks of 6 hours and 1.5 hours, due to the dip experienced around 2pm and the heat experienced over the midday period, and for societies which already have an established culture of napping, for example in Asia, albeit many cultures are now discussing and highlighting the importance of the practise and many large companies in the West provide appropriate spaces for their employees to take a nap.

The present invention seeks to provide a product which is able to obviate the above-referenced problems and provide an enhanced napping experience or improved period of quiet.

The invention is defined in the appended claims 1 to 13.

The invention will now be more particularly described, by way of example only, with reference to the accompanying drawings, in which:
Figure 1 shows a representation of an ear-and-eye mask in accordance with an embodiment of the invention;
Figure 2 shows a diagrammatic representation of an ear device ; and
Figure 3 shows a diagrammatic representation of an ear device with external controller and wireless communication in accordance with the an embodiment of the invention

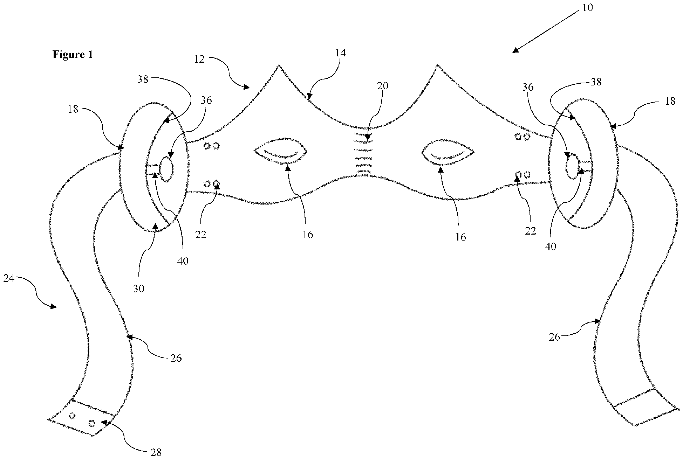

Referring to Figure 1, there is shown an ear-and-eye mask, indicated globally at 10 which is suitable for wearing by a user in order to create an effective napping environment.

The ear-and-eye mask 10 comprises a mask body 12 having an eye mask 14, preferably comprising a pair of eye coverings 16 positioned to in-use cover a user's eyes, and a pair of ear coverings 18 positioned to in-use cover a user's ears. It is preferred that the eye coverings 16 are ergonomically shaped to a user's eye sockets for comfort, but it will be equally apparent that a uniform mask portion could be provided which covers both eyes simultaneously. Each eye covering 16 can be recessed to avoid smudging make-up.

It may be possible that the eye mask 14 includes one or more indicia portions, which may be illuminable, for example, to highlight a branding of the ear-and-eye mask 10 in use.

The eye mask 14 is preferably formed from a soft material which can comfortably rest against the user's face, such as cotton, foam padding, silk, or similar material, which may be breathable for heat control, and may include one or more adjustment means for modifying a shape and/or fit of the eye mask 14 to a specific user. For example, stretchable fabric may be provided at or adjacent to a nose bridge region 20 of the eye mask 14, and/or magnetic clasps 22, preferably four magnetic clasps to permit size adjustability, may be provided to allow for adjustment of the width of the eye mask 14. The eye coverings 16 and/or nose bridge region 20 may be detachable from the sleep mask body 12 for easy cleaning.

The ear-and-eye mask 10 includes attachment means for demountably attaching to a user, and in the depicted embodiment, this is provided as a strap 24 which is engagable around the rear of a user's head. The strap 24 may preferably comprise a pair or strap portions 26 which are receivably engagable with one another, for example, via a releasable fastener 28 such as a magnetic clasp, hook and loop fastener, or popper. This allows for ready engagement of the ear-and-eye mask 10 onto a user's head. This provides room for a few centimetres adjustment, whilst the side and rear fasteners may allow for approximately six centimetres adjustability, which is suitable for covering standard head sizes for men and women. It is, however, possible that no strap 24 is provided, and instead the ear-and-eye mask 10 hooks over a user's ears so as to avoid tangling the user's hair.

The eye mask 14 provides a mechanism for reducing or eliminating the exposure of a user to light which can be detrimental to napping. Darkness triggers the release of melatonin, the sleep hormone which prepares the body for sleep. The ear coverings 18 are then designed to reduce or eliminate the exposure of the user to external noises, attempting to best replicate silence for optimum napping potential.

Each ear covering 18 comprises an outer cup 30 which can cup or enclose a user's ear when the ear-and-eye mask 10 is worn, and comprises a first passive noise-dampening member 31 which is capable of acoustically dampening noise from external sources, and may be formed from plastics materials, foam, insulators, or any appropriately resistant material. The outer cup 30 may have a perimeter wall 32 forming an inner cup which defines an enclosure 34, cavity or chamber therein, with the outer cup 30 defining a cover for the user's ear.

In a preferred embodiment, the speaker 36 is mounted at the base of the support stem 40 forming an audio isolator. The support stem 40 creates a mushroom-like shape, having a hollow stalk which extends into the ear canal of the user which acts as an acoustic seal to further prevent unwanted noise from entering into the ear canal. The support stem 40 could be omitted, however, and a more traditional earphone design utilised when the speaker positioned in the enclosure is coaxially positioned relative to the audio capture device to hear what the speak emits; The speaker 36 could, however, be mounted anywhere within the support stem 40, including but not limited to, the tip of the stem resting in the aural cavity.

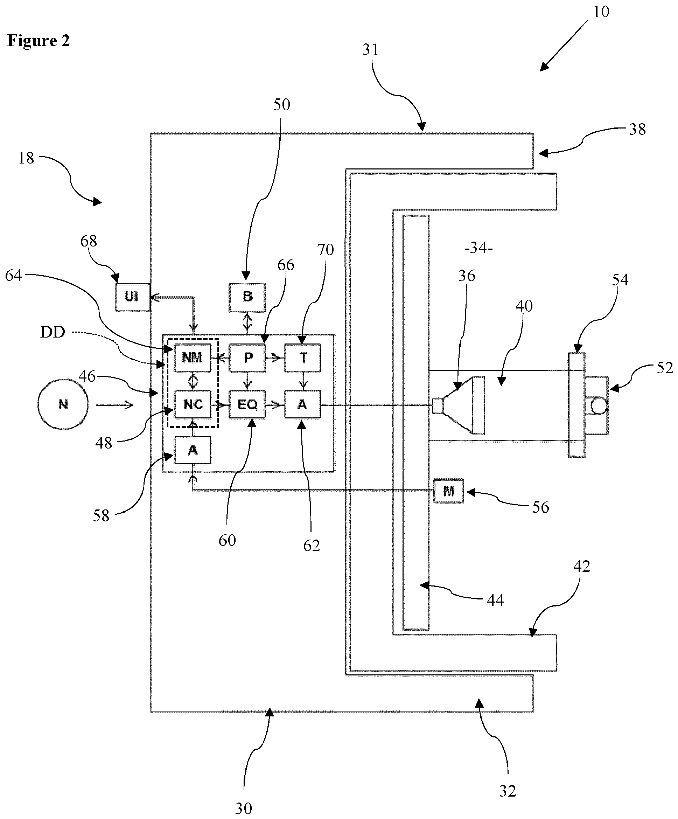

A more detailed representation of the ear device 18 is shown in Figure 2, in which a second passive noise-dampening member 42 can be seen which is positioned in the enclosure 34 of the outer cup 30, received within the perimeter wall 32 and at least in contact with the rim 38 of the outer cup 30. The second passive noise-dampening member 42 is preferably formed from a material which is a poor transmitter of noise, such as foam or padding. Such an ear covering 18, with the second passive noise-dampening member 42 being constructed from high-quality sound dampening materials is particularly effective at reducing higher frequencies. This may also have the advantage of being comfortable for a user's ear when the ear-and-eye mask 10 is worn. The provision of the support stem 40 also provides a locator within the ear canal, ensuring that the speaker 36 is located in the correct position for optimum noise attenuation, and also limiting the prospect of the speaker 36 falling out of the ear canal, which would otherwise be an issue for ear buds.

Preferably, there is provided a biasing element 44, such as a planar spring, which allows for adjustment of the support stem 40 to fit the speaker 36 into the correct location in the ear canal of the user. The planar spring provides some degree of flexibility of the position of the support stem 40, and can serve to sandwich the second passive noise-dampening member 42 in the enclosure 34.

The ear device 18 includes a noise-attenuation and generation assembly 46, which may be located within the support stem 40, though it will be appreciated that the electronic components could in fact, and may preferably, be positioned in the outer cup 30 as illustrated. At least part of the noise-attenuation-and-generation assembly 46 is provided in the digital domain DD, as opposed to the analogue domain, the noise cancelling circuit 48 and the noise masking circuit 64. The noise-attenuation and generation assembly 46 assists with the creation of silence or near-silence for the user. This is achieved by the passively attenuated signal passing in to the digital domain where it is processed and reproduced using a noise cancelling circuit 48 which communicates with the adaptive noise spectral analyser and generator 64 to generate a masking noise of the residual signal for output by the speaker 36 to mask noise external to the ear-and-eye mask 10. Preferably the noise-attenuation and generation assembly 46 is powered by an onboard power supply 50, such as a rechargeable battery, which may allow the ear-and-eye mask 10 to be used in a wireless context. Whilst the present ear-and-eye mask 10 is intended for napping purposes, it is preferred that the power supply 50 be capable of providing power for at least the duration of an extended sleep, that is, for upwards of six hours if fully charged. A wireless power supply could also be considered, as an alternative. The speaker 36 may be provided on the end of the support stem 40 so as to have an ergonomic tip 52, which may rest comfortably in the user's inner ear, and which may have a replaceable bud or may be easily wipeable or cleanable. A projecting lip or rim 54 may also be provide which acts as an inner seal for the ear.

In the invention, there is provided a microphone or similar audio capture device 56 which is able to receive the audio input to be attenuated and reproduced. This is preferably positioned inside at the base of the support stem 40 such that external sounds are attenuated by both the first and second passive noise dampening members 31, 42 before reaching the audio capture device 56. Preferably, the audio capture device 56 is connected to an amplifier 58 to permit amplification of the noise captured, prior to digital processing and generation. In a most preferable embodiment of the invention, the unwanted residual signal is analysed before generating a suitable signal to eliminate as much, if not all, of the unwanted residual signal by modification of the masking noise.

In this arrangement, the noise cancelling circuit 48 is preferably connected to an equalizer 60, that is, a device capable of adjusting the balance between frequency components within an electronic signal. The output of the noise cancelling circuit 48 and noise masking circuit 64 from the equalizer 60 may proceed through a secondary amplifier 62, before being transmitted to the speaker 36 for transmission to the user.

The noise cancelling circuit 48 may be associated with a noise masking circuit 64 which communicate back and forth in the digital domain to generate the most appropriate masking noise. It will be apparent that the noise cancelling circuit 48 and noise masking circuit 64 could be co-located on a single processor. The noise masking circuit 64 may advantageously utilise adaptive spectral noise analysis and generation, which may be a physical device or may be provided as control software implemented on a processor of the noise masking circuit 64, or indeed any other component of the noise-attenuation-and-generation assembly 46, by application of an adaptive spectral noise analyser and generator algorithm which dynamically compensates frequencies and/or decibel levels in order to compensate for and minimise the disturbing effect of external noise which could still be heard by the user after passive attenuation and cancellation by the noise cancelling circuit 48. This advance allows for dynamic noise attenuation.

The noise masking circuit 64 in combination with the noise cancelling circuit 48 may be able to dynamically adjust frequency and/or volume levels, in conjunction with the equalizer 60 where required in order to achieve an effect which is as close to silence as is feasible within the ear device 18.

The noise-attenuation and generation assembly 46 actively attenuates the residual signal that passes through the passive noise-dampening member 42. The noise cancellation circuit 48 in combination with the noise masking circuit 64 using dynamic noise spectral analysis assesses the left-over unwanted signal following passive noise-dampening, and after assessing the decibel and frequency level of the signal, and allowing for the cancellation by the noise cancelling circuit 48 digitally manufactures an appropriate signal to mask it.

In addition to dynamic masking of an input noise, it may also be possible to provide a personalisation circuit 66 which allows for user-specific modifiers to be applied to the masking noise. For example, in order to achieve an optimised noise attenuation, it may be preferred that, for a specific user, certain frequencies are boosted or cut, based on their hearing range. There may be a dedicated user interface 68 which is positioned on the mask body 12 which allows the user to directly affect the personalisation circuit 66 for an improved napping experience. The personalisation settings may be input at set-up of the ear device 18, and may stay fixed until the user wishes to change it either due to ageing or wishing to lend the product to a third party. For example, the user may be able to tap the outer cup 30, via the user interface 68, in order to deactivate the functionality of the ear-and-eye mask 10 if disturbed.

Primarily, the support stem 40 serves to act as an audio-isolator between the speaker 36 and the audio capture device 56. The support stem 40 also serves as a locator for the speaker 36 in the ear to limit dislodgment. In use, therefore, the user is able to wear the ear-and-eye mask 10 to cover their eyes with the eye mask 14, and their ears with the ear coverings 18. Light is blocked by the eye mask 14, and external noises are attenuated by the passive noise-dampening member 42 of the ear coverings 18. The attenuated noise can then be further obviated by the noise-attenuation and generation assembly 46, which reduces the disturbances further.

A user may be able to set a period of noise attenuation via a timer 70 of the noise-attenuation and generation assembly 46, which may be alterable via the user interface 68. A preferred default timing may be of the order of twenty minutes, for a short duration napping period which is beneficial to the user's health. However, the timer 70 can be configured to deactivate the masking noise after a predetermined or user-settable duration. The user interface 68 may comprise, for example, an activation switch, such as an on/off button, and may have activatable timer settings. Where a timer 70 is used, it may be possible to provide a dedicated wake-up sound which is a cue for the user to wake up from their nap. Similarly, an introductory or lead-in period to the silence created by the noise-attenuation and generation assembly 46 could be provided, allowing the user a period to sit and relax prior to their nap.

It is also noted that several studies have noted that if light, particularly blue light, is shone into the ear canal, this can result in a measurable response in the brain, as measured via EEG. This can be used in the mitigation of the effects of jetlag. In order to enhance the post-nap rousing routine, it may therefore be viable to provide an illumination element, preferably in a wavelength range corresponding with blue light, which is aligned to illuminate the ear canal. This could for example be provided as an illumination element on the support stem 40, where provided.

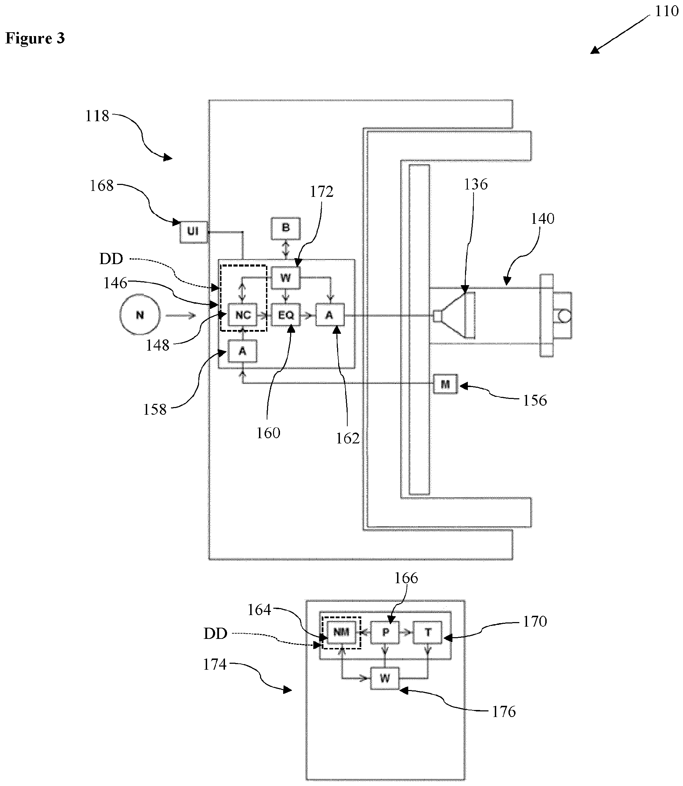

An alternative embodiment of the ear device is illustrated in Figure 3, in which the ear-and-eye mask is indicated globally at 110. Identical or similar reference numerals will be used to refer to identical or similar reference numerals to those used in the above-described embodiment, and further detailed description is omitted for brevity.

Onboard the ear device 118, the noise-attenuation assembly 146 is split between the ear device and an external controller. The ear device 118 may comprise the audio capture device 156 feeding the amplifier 158 which enters the digital domain and the noise cancellation circuit 148. The noise attenuation assembly 146 communicates wirelessly with the adaptive spectral noise analyser and generator in the noise masking circuit 164 and the noise attenuation and generation assembly is connected to the equalizer 160, and onward to the secondary amplifier 162, so that the resultant signal of their communications is output via the speaker 136 of the ear covering 118, via the support stem 140.

However, instead of an onboard noise attenuation and generating assembly, the digital noise cancelling circuit 148 is coupled to a wireless communicator 172 which is adapted to receive control commands for the noise-attenuation and generation assembly 146 from an external device 174, such as a tablet computer or smartphone having a corresponding control application on which may be positioned the noise masking circuit 164 with adaptive spectral analysis and generation.

The external device 174 has a corresponding wireless communicator 176 which may be in communication with any or all of a noise masking circuit 164, a personalisation circuit 166, or a timer 170, thereby allowing the user to control the functionality of the ear-and-eye mask 110 without needing to remove the ear-and-eye mask 110 and also allowing the possibility to interact with other apps that track the user's health and wellness. Indeed, the ear-and-eye mask 110 is configured so as to be communicable with a variety of external computer applications, which may be of assistance with improving the napping experience.

The control application on the external device 174 may also allow for control over other devices; for example, it may be possible for the user to alter settings on other external devices, such as a smartphone, by the use of a do-not-disturb profile or similar which corresponds with the nap settings applied to the ear-and-eye mask 110. The control application could provide a number of other linked benefits for the user, for example, a choice of masking noises could be provided via the user interface 168, and the control application could link to a user's diary, so that nap times may be scheduled. A number of additional nap minutes or similar timing mechanism could be provided via the control application, to permit additional user control, or to provide a visual indication to third parties of a remaining nap time. Community napping aspects could also be introduced, via social connections to other users.

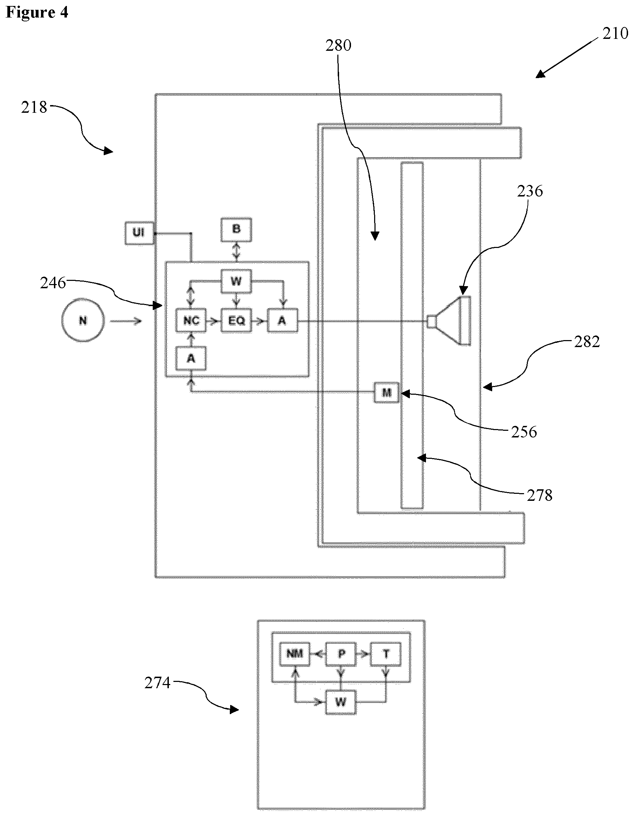

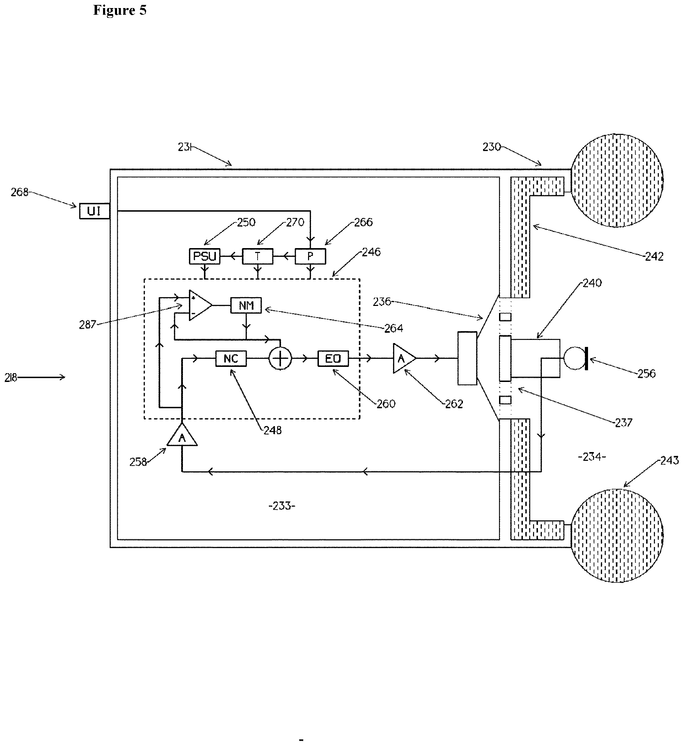

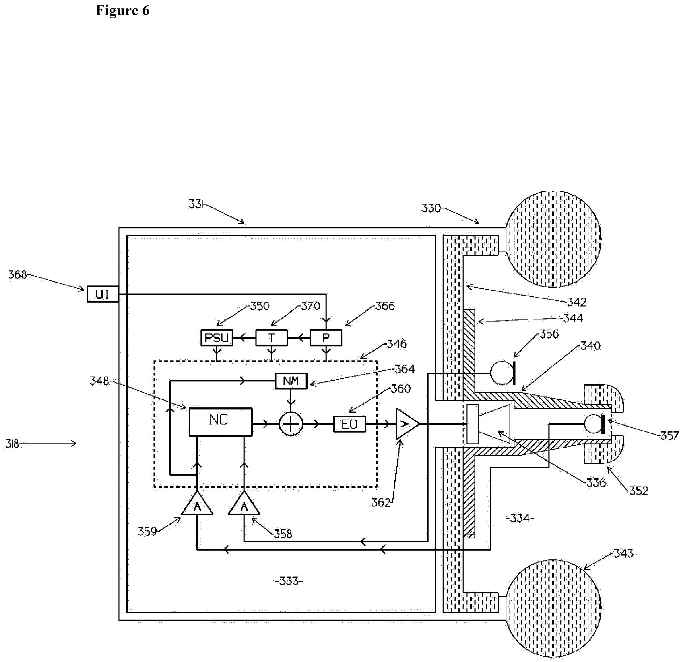

A further alternative embodiment of the ear device is illustrated in Figure 4, in which the ear-and-eye mask is indicated globally at 210. Identical or similar reference numerals will be used to refer to identical or similar reference numerals to those used in the above-described embodiment, and further detailed description is omitted for brevity.

As with the previous embodiments, the noise-attenuation-and-generation assembly 246 may be provided inside the ear coverings 218, and may be communicable with an external device 274. However, the speaker 236 to audio capture device 256 architecture is altered. It will be apparent, however, that the fully integrated noise-attenuation-and-generation assembly 246 as shown in Figure 2 could just as easily be applied to the present embodiment.

Instead of the speaker 236 being positioned at the base of a stem, there is provided a, preferably planar, audio isolator, such as an audio isolator plate 278 which acts as a baffle between the speaker 236 and the audio capture device 256. The audio capture device 256 is therefore positioned within its own self-contained chamber 280, and therefore the audio capture device 256 only transmits the attenuated signal to the noise-attenuation-and-generation assembly 246. Once the signal has been processed by the adaptive spectral analyser and generator, the compensation frequency and/or volume for countering this unwanted remnant signal following noise cancellation then being transmitted to the speaker 236 for output to the user. Since no stem is provided in this embodiment of the invention, a traditional mesh fabric 282 or similar protective member may be provided to conceal the speaker 236.

In this embodiment in which the support stem is removed, and the overall ear covering 218 design is closer to that of traditional headphones, the speaker 236 and audio capture device 256 follow exactly the same topology, even though the physical position of the audio capture device 256 is now located behind the speaker 236. The speaker 236 remains isolated from the audio capture device 256, since there is a solid element between the audio capture device 256 and the speaker 2367, which provides physical acoustic isolation. The audio capture device 256 is mounted within the chamber 280 in order to pick up residual noise that first stage passive attenuation has failed to eliminate. In turn, this is fed into the signal processing electronics of the noise-attenuation-and-generation assembly 246 for algorithmic processing and masking. In general, it is therefore possible to provide an improved mechanism for creating silence through an ear covering device comprising: a device body having at least one ear covering 18 positioned to in-use cover a user's ears; the or each ear covering 18 including an outer cup 30 including at least one passive noise dampening member and an inner cup which in use define as enclosure; an audio capture device 56 which positioned in the enclosure ; and a noise-attenuation-and-generation assembly 46 comprising: a digital processing domain 48, 64 where the signal is attenuated processed and reproduced at a suitable compensation frequency and/or volume in order to counter the input from the audio capture device 56 following passive noise attenuation; and a speaker 36 positioned in the enclosure 34 or isolated in a stem 40 apart from the audio capture device 56 with optionally in this case a further audio capture device being positioned to hear what the speaker emits, a signal from the noise-attenuation and generation assembly being output by the speaker 36 to the user's ears.

Alternatively, it could be considered that the present disclosure relates to an ear-and-eye mask 10 comprising a noise-attenuation-and-generation assembly 46 having a digital domain, the noise-attenuation-and-generation assembly comprising, in the digital domain, a noise cancelling-circuit 48 and a noise masking circuit 64, wherein the noise-cancelling circuit 48 receives an input from an audio capture device 56 thereof which it communicates with the noise masking circuit 64 An adaptive spectral noise analyser and generator is provided by the noise masking circuit 64 which determines the frequency and/or volume after noise cancellation, and wherein the noise attenuation-and-generation assembly 46 through the use of the spectral analyser and generator of the noise masking circuit 64 then determines and generates a suitable compensation frequency and/or volume for countering this unwanted residual signal following noise attenuation by the noise cancellation circuit 58 for output by a speaker 36 to the user's ears.

By providing an additional audio capture device 56 inside the enclosure, which is spaced apart from the speaker 36, noise attenuation can proceed as normal, but the additional audio capture device hears what the speaker emits. This signal can be analysed in the digital domain by the noise attenuation and generation assembly which allows for the creation of a digitally manufactured signal which compensates for the unwanted remnant signal, effectively creating dynamic noise attenuation to create silence.

It may also be possible to provide a, preferably analogue, compressor as part of a noise-attenuation assembly which is capable of capping sudden interruptions of sound across the frequency spectrum. This would effectively create an analogue equivalent of the digital solution previously described.

It is therefore possible to provide an ear-and-eye mask which is able to permit napping from 10 to 90 minutes, excluding noise and light from the user during this period to ensure that a restful environment can be readily created.

The words 'comprises/comprising' and the words 'having/including' when used herein with reference to the present invention are used to specify the presence of stated features, integers, steps or components, but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The embodiments described above are provided by way of examples only, and various other modifications will be apparent to persons skilled in the field without departing from the scope of the invention as defined herein. The invention is defined by the appended claims.

## Claims

1. An ear device (10; 110; 210) comprising:
at least one ear covering (18; 118; 218) positioned to in-use cover a user's ears, the or each ear covering (18; 118; 218) including an outer cup (30) comprising at least one passive noise dampening member (31, 42) and an inner cup which in use define an enclosure;
an audio capture device (56; 156; 256) which is positioned in the enclosure;
a noise-attenuation-and-generation assembly (46; 146; 246) which comprises a noise cancelling circuit (48; 148; 248) and a noise masking circuit (64; 164; 264);
the noise-cancelling circuit (48; 148; 248) receiving an input from the audio capture device (56; 156; 256) and producing a signal;
the noise masking circuit (64; 164; 264) comprising an adaptive spectral noise analyser and generator which determines and generates a compensation frequency or a compensation frequency and volume to mask a residual signal; and
a speaker (36; 136; 236) positioned in the enclosure and/or a support stem thereof;
**characterized in that**
the signal from the noise cancelling circuit (48; 148; 248) is communicated to the noise masking circuit (64; 164; 264), so that the noise masking circuit determines the residual signal after attenuation by the noise-cancelling circuit (48; 148; 248) and the adaptive spectral noise analyser and generator determines the frequency or the frequency and volume of the residual signal and based on this frequency or frequency and volume of the residual signal dynamically generates a suitable compensation frequency or a compensation frequency and volume to mask the residual signal which is sent to the speaker alongside the signal from the noise cancelling circuit (48; 148; 248) to be output by the speaker (36; 136; 236) to the user's ears in order to counter the input from the audio capture device.

2. An ear device (18; 118; 218; 318) as claimed in claim 1, wherein noise-attenuation-and-generation assembly (46; 146; 246) comprises an amplifier (58; 158) and an equalizer (60; 160) and a secondary amplifier (62; 162).

3. An ear device (18; 118; 218; 318) as claimed in claim 1 or claim 2, wherein the audio capture device (56; 156; 256) is not isolated from the speaker (36; 136; 236) and is positioned adjacently or coaxially next to the speaker (36; 136; 236) in order to hear the output of the speaker (36; 136; 236).

4. An ear device (18; 118; 218; 318) as claimed in any one of the claims 1 or 2, wherein the or a further audio capture device is isolated from the speaker.

5. An ear device (18; 118; 218; 318) as claimed in any one of the preceding claims, wherein the noise-attenuation-and-generation assembly (46; 146; 246; 346) comprises a personalisation circuit (66; 166), the personalisation circuit (66; 166) being adapted to receive a user input to modify the signal from the noise-attenuation-and-adaptive-generation assembly (46; 146; 246).

6. An ear device (18; 118; 218; 318) as claimed in claim 5, wherein the personalisation circuit applies user-specific modifiers to boost or cut the compensation frequency, based on the user's hearing range.

7. An ear device (18; 118; 218; 318) as claimed in any one of the preceding claims, further comprising a timer (70; 170; 270; 370) associated with the noise-attenuation-and generation assembly (46; 146; 246; 346), the timer (70; 170; 270; 370) being configured to deactivate the signal from the noise-attenuation-and-generation assembly (46; 146; 246) after a predetermined or user-settable duration.

8. An ear device (18; 118; 218; 318) as claimed in any one of the preceding claims, further comprising an external computing device which may have a wireless communicator (172), which allows the noise-attenuation-and-generation assembly (146; 246) to be split between an external device (174; 274) and the ear device.

9. An ear device (18; 118; 218; 318) as claimed in any one of the preceding claims, further comprising a user interface (68; 168) on the ear device or an external controller to permit the user control of the noise-attenuation and generation assembly (46; 146; 246; 346), and allow the user to alter settings and affect the personalisation circuit.

10. An ear-device (18; 118; 218; 318), as claimed in any one of the preceding claims, further comprising an onboard power supply (50) which is configured to power at least the noise-attenuation assembly (46; 146; 246).

11. An ear device (18; 118; 218; 318), as claimed in any one of the preceding claims wherein the adaptive spectral noise analyser and generator of the noise masking circuit (64; 164; 264; 364) utilizes an algorithm/s.

12. An ear device (18; 118; 218; 318) as claimed in any one of the preceding claims which is part of an ear-and-eye mask (10).

13. A method of creating enhanced napping conditions for a user, the method comprising the steps of providing an ear device as claimed in any one of the preceding claims, and when a napping condition or period of quiet is desired, the user wears the ear device.

## Patentansprüche

1. Ohrvorrichtung (10; 110; 210), umfassend:
mindestens eine Ohrabdeckung (18; 118; 218), die positioniert ist, um im Gebrauch die Ohren eines Benutzers abzudecken, wobei die oder jede Ohrabdeckung (18; 118; 218) eine äußere Schale (30), die mindestens ein passives Geräuschdämmungselement (31; 42) umfasst, und eine innere Schale beinhaltet, die im Gebrauch ein Gehäuse definieren;
eine Audioerfassungsvorrichtung (56; 156; 256), die in dem Gehäuse positioniert ist;
eine Geräuschdämpfungs- und -erzeugungsanordnung (46; 146; 246), die eine Geräuschunterdrückungsschaltung (48; 148; 248) und eine Geräuschmaskierungsschaltung (64; 164; 264) umfasst;
wobei die Geräuschunterdrückungsschaltung (48; 148; 248) einen Eingang von der Audioerfassungsvorichtung (56; 156; 256) empfängt und ein Signal produziert;
wobei die Geräuschmaskierungsschaltung (64; 164; 264) einen adaptiven Analysator und Generator für spektrales Rauschen umfasst, der eine Kompensationsfrequenz und ein Kompensationsvolumen bestimmt und erzeugt, um ein Restsignal zu maskieren; und
einen Lautsprecher (36; 136; 236), der in dem Gehäuse und/oder einem Trageschaft davon positioniert ist;
**dadurch gekennzeichnet, dass** das Signal von der Geräuschunterdrückungsschaltung (48; 148; 248) an die Geräuschmaskierungsschaltung (64; 164; 264) kommuniziert wird, so dass die Geräuschmaskierungsschaltung das Restsignal nach einer Dämpfung durch die Geräuschunterdrückungsschaltung (48; 148; 248) bestimmt und der adaptive Analysator und Generator für spektrales Rauschen die Frequenz oder die Frequenz und das Volumen des Restsignals bestimmt und eine geeignete Kompensationsfrequenz oder eine geeignete Kompensationsfrequenz oder ein geeignetes Kompensationsvolumen dynamisch erzeugt, um das Restsignal zu maskieren, das an den Lautsprecher neben dem Signal von der Geräuschunterdrückungsschaltung (48; 148; 248) gesendet wird, um von dem Lautsprecher (36; 136; 236) an die Ohren des Benutzers ausgegeben zu werden, um dem Eingang von der Audioerfassungsvorrichtung entgegenzuwirken.

2. Ohrvorrichtung (18; 118; 218; 318) nach Anspruch 1, wobei die Geräuschdämpfungs- und -erzeugungsanordnung (46; 146; 246) einen Verstärker (58; 158) und einen Entzerrer (60; 160) und einen sekundären Verstärker (62; 162) umfasst.

3. Ohrvorrichtung (18; 118; 218; 318) nach Anspruch 1 oder Anspruch 2, wobei die Audioerfassungsvorrichtung (56; 156; 256) von dem Lautsprecher (36; 136; 236) nicht isoliert ist und angrenzend an oder koaxial neben dem Lautsprecher (36; 136; 236) positioniert ist, um den Ausgang des Lautsprechers (36; 136; 236) zu hören.

4. Ohrvorrichtung (18; 118; 218; 318) nach einem der Ansprüche 1 oder 2, wobei die oder eine weitere Audioerfassungsvorrichtung von dem Lautsprecher isoliert ist.

5. Ohrvorrichtung (18; 118; 218; 318) nach einem der vorhergehenden Ansprüche, wobei die Geräuschdämpfungs- und -erzeugungsanordnung (46; 146; 246; 346) eine Personalisierungsschaltung (66; 166) umfasst, wobei die Personalisierungsschaltung (66; 166) dazu eingerichtet ist, einen Benutzereingang zu empfangen, um das Signal von der Geräuschdämpfungs- und adaptiven Geräuscherzeugungsanordnung (46; 146; 246) zu modifizieren.

6. Ohrvorrichtung (18; 118; 218; 318) nach Anspruch 5, wobei die Personalisierungsschaltung benutzerspezifische Modifikatoren anwendet, um die Kompensationsfrequenz auf der Basis des Hörbereichs des Benutzers anzuheben oder abzusenken.

7. Ohrvorrichtung (18; 118; 218; 318) nach einem der vorhergehenden Ansprüche, weiterhin umfassend einen Timer (70; 170; 270; 370), der mit der Geräuschdämpfungs- und -erzeugungsanordnung (46; 146; 246; 346) assoziiert ist, wobei der Timer (70; 170; 270; 370) dazu konfiguriert ist, das Signal von der Geräuschdämpfungs- und -erzeugungsanordnung (46; 146; 246) nach einer vorbestimmten oder vom Benutzer einstellbaren Dauer zu deaktivieren.

8. Ohrvorrichtung (18; 118; 218; 318) nach einem der vorhergehenden Ansprüche, weiterhin umfassend eine externe Datenverarbeitungsvorrichtung, die einen drahtlosen Kommunikator (172) aufweisen kann, der ermöglicht, dass die Geräuschdämpfungs- und -erzeugungsanordnung (146; 246) zwischen einer externen Vorrichtung (174; 274) und der Ohrvorrichtung aufgeteilt wird.

9. Ohrvorrichtung (18; 118; 218; 318) nach einem der vorhergehenden Ansprüche, weiterhin umfassend eine Benutzeroberfläche (68; 168) auf der Ohrvorrichtung oder einer externen Steuereinheit, um dem Benutzer eine Steuerung der Geräuschdämpfungs- und -erzeugungsanordnung (46; 146; 246; 346) zu ermöglichen und dem Benutzer zu ermöglichen, Einstellungen zu ändern und auf die Personalisierungsschaltung einzuwirken.

10. Ohrvorrichtung (18; 118; 218; 318) nach einem der vorhergehenden Ansprüche, weiterhin umfassend eine integrierte Energieversorgung (50), die dazu konfiguriert ist, mindestens die Geräuschdämpfungsanordnung (46; 146; 246) mit Energie zu versorgen.

11. Ohrvorrichtung (18; 118; 218; 318) nach einem der vorhergehenden Ansprüche, wobei der adaptive Analysator und Generator für spektrales Rauschen der Geräuschmaskierungsschaltung (64; 164; 264; 364) einen oder mehrere Algorithmen nutzt.

12. Ohrvorrichtung (18; 118; 218; 318) nach einem der vorhergehenden Ansprüche, die Teil einer Ohren- und Augenmaske (10) ist.

13. Verfahren zur Erzeugung von verbesserten Schlummerbedingungen für einen Benutzer, wobei das Verfahren die Schritte eines Bereitstellens einer Ohrvorrichtung nach einem der vorhergehenden Ansprüche umfasst, und der Benutzer die Ohrvorrichtung trägt, wenn eine Schlummerbedingung oder eine Ruheperiode gewünscht wird.

## Revendications

1. Dispositif d'oreille (10 ; 110 ; 210) comprenant :
au moins une couverture d'oreille (18 ; 118 ; 218) positionnée pour couvrir en cours d'utilisation les oreilles d'un utilisateur, la ou chaque couverture d'oreille (18 ; 118 ; 218) incluant une coupelle externe (30) comprenant au moins un élément d'amortissement de bruit passif (31, 42) et une coupelle interne qui en cours d'utilisation définissent une enceinte ;
un dispositif de capture audio (56 ; 156 ; 256) qui est positionné dans l'enceinte ;
un ensemble d'atténuation et de génération de bruit (46 ; 146 ; 246) qui comprend un circuit d'annulation de bruit (48 ; 148 ; 248) et un circuit de masquage de bruit (64 ; 164 ; 264) ;
le circuit d'annulation de bruit (48 ; 148 ; 248) recevant une entrée en provenance du dispositif de capture audio (56 ; 156 ; 256) et produisant un signal ;
le circuit de masquage de bruit (64 ; 164 ; 264) comprenant un analyseur et générateur de bruit spectral adaptatif
qui détermine et génère une fréquence de compensation ou une fréquence et un volume de compensation pour masquer un signal résiduel ; et
un haut-parleur (36 ; 136 ; 236) positionné dans l'enceinte et/ou une tige de support de celle-ci ;
**caractérisé en ce que** le signal provenant du circuit d'annulation de bruit (48 ; 148 ; 248) est communiqué au circuit de masquage de bruit (64 ; 164 ; 264), de sorte que le circuit de masquage de bruit détermine le signal résiduel après atténuation par le circuit d'annulation de bruit (48 ; 148 ; 248) et l'analyseur et générateur de bruit spectral adaptatif détermine la fréquence ou la fréquence et le volume du signal résiduel et en fonction de cette fréquence ou de cette fréquence et de ce volume du signal résiduel génère dynamiquement une fréquence de compensation appropriée ou une fréquence et un volume de compensation pour masquer le signal résiduel qui est envoyé au haut-parleur à côté du signal provenant du circuit d'annulation de bruit (48 ; 148 ; 248) devant être délivré en sortie par le haut-parleur (36 ; 136 ; 236) aux oreilles de l'utilisateur afin de contrer l'entrée provenant du dispositif de capture audio.

2. Dispositif d'oreille (18 ; 118 ; 218 ; 318) selon la revendication 1, dans lequel l'ensemble d'atténuation et de génération de bruit (46 ; 146 ; 246) comprend un amplificateur (58 ; 158) et un égaliseur (60 ; 160) et un amplificateur secondaire (62 ; 162).

3. Dispositif d'oreille (18 ; 118 ; 218 ; 318) selon la revendication 1 ou la revendication 2, dans lequel le dispositif de capture audio (56 ; 156 ; 256) n'est pas isolé du haut-parleur (36 ; 136 ; 236) et est positionné de manière adjacente ou coaxiale à côté du haut-parleur (36 ; 136 ; 236) afin d'entendre la sortie du haut-parleur (36 ; 136 ; 236).

4. Dispositif d'oreille (18 ; 118 ; 218 ; 318) selon l'une quelconque des revendications 1 ou 2, dans lequel le dispositif de capture audio ou un autre dispositif de capture audio est isolé du haut-parleur.

5. Dispositif d'oreille (18 ; 118 ; 218 ; 318) selon l'une quelconque des revendications précédentes, dans lequel l'ensemble d'atténuation et de génération de bruit (46 ; 146 ; 246 ; 346) comprend un circuit de personnalisation (66 ; 166), le circuit de personnalisation (66 ; 166) étant conçu pour recevoir une entrée utilisateur pour modifier le signal provenant de l'ensemble d'atténuation et de génération adaptative de bruit (46 ; 146 ; 246).

6. Dispositif d'oreille (18 ; 118 ; 218 ; 318) selon la revendication 5, dans lequel le circuit de personnalisation applique des modificateurs spécifiques de l'utilisateur pour renforcer ou couper la fréquence de compensation, en fonction de la plage d'audition de l'utilisateur.

7. Dispositif d'oreille (18 ; 118 ; 218 ; 318) selon l'une quelconque des revendications précédentes, comprenant en outre un temporisateur (70 ; 170 ; 270 ; 370) associé à l'ensemble d'atténuation et de génération de bruit (46 ; 146 ; 246 ; 346), le temporisateur (70 ; 170 ; 270 ; 370) étant configuré pour désactiver le signal provenant de l'ensemble d'atténuation et de génération de bruit (46 ; 146 ; 246) après une durée prédéterminée ou réglable par l'utilisateur.

8. Dispositif d'oreille (18 ; 118 ; 218 ; 318) selon l'une quelconque des revendications précédentes, comprenant en outre un dispositif informatique externe qui peut avoir un système de communication sans fil (172), qui permet à l'ensemble d'atténuation et de génération de bruit (146 ; 246) d'être divisé entre un dispositif externe (174 ; 274) et le dispositif d'oreille.

9. Dispositif d'oreille (18 ; 118 ; 218 ; 318) selon l'une quelconque des revendications précédentes, comprenant en outre une interface utilisateur (68 ; 168) sur le dispositif d'oreille ou un organe de commande externe pour permettre la commande par l'utilisateur de l'ensemble d'atténuation et de génération de bruit (46 ; 146 ; 246 ; 346), et permettre à l'utilisateur de modifier des réglages et d'influencer le circuit de personnalisation.

10. Dispositif d'oreille (18 ; 118 ; 218 ; 318), selon l'une quelconque des revendications précédentes, comprenant en outre une alimentation en puissance intégrée (50) qui est configurée pour alimenter en puissance au moins l'ensemble d'atténuation de bruit (46 ; 146 ; 246).

11. Dispositif d'oreille (18 ; 118 ; 218 ; 318), selon l'une quelconque des revendications précédentes dans lequel l'analyseur et générateur de bruit spectral adaptatif du circuit de masquage de bruit (64 ; 164 ; 264 ; 364) utilise un/des algorithme/s.

12. Dispositif d'oreille (18 ; 118 ; 218 ; 318) selon l'une quelconque des revendications précédentes qui fait partie d'un masque pour les oreilles et les yeux (10).

13. Procédé de création de meilleures conditions de sieste pour un utilisateur, le procédé comprenant les étapes de fourniture d'un dispositif d'oreille selon l'une quelconque des revendications précédentes, et lorsqu'une condition de sieste ou une période de tranquillité est souhaitée, l'utilisateur porte le dispositif d'oreille.
